(19) Europäisches Patentamt
European Patent Office
Office européen des brevets

(11) Publication number: **0 142 164 B1**

(12) # EUROPEAN PATENT SPECIFICATION

(45) Date of publication of patent specification: **12.07.89**

(51) Int. Cl.[4]: **G 01 N 33/20**, G 01 N 31/22

(21) Application number: **84113694.8**

(22) Date of filing: **13.11.84**

(54) **Method for detecting phosphorus in metallic material.**

(30) Priority: **14.11.83 JP 213497/83**
**24.07.84 JP 153799/84**

(43) Date of publication of application:
**22.05.85 Bulletin 85/21**

(45) Publication of the grant of the patent:
**12.07.89 Bulletin 89/28**

(84) Designated Contracting States:
**DE FR GB IT SE**

(56) References cited:
**DE-C- 873 319**
**US-A-3 796 543**

**CHEMICAL ABSTRACTS, vol. 26, 1932, page 5871, Columbus, Ohio, US; M. NIESSNER: "New methods for identifying minor constituents of alloys and for detecting segregation in metal-working materials" & MIKROCHEMIE 12, 1-24(1932)**

**CHEMICAL ABSTRACTS, vol. 41, 1947, column 6171 d-f, Columbus, Ohio, US; H.L. KATZ et al.: "Direct colorimetric method for phosphorus in all types of steel" & ANAL. CHEM. 19, 612-14(1947)**

(73) Proprietor: **KAWASAKI STEEL CORPORATION**
**No. 1-28, 1-Chome Kitahonmachi-Dori**
**Chuo-Ku, Kobe-Shi Hyogo 651 (JP)**

(72) Inventor: **Funahashi, Yoshiko c/o Research Laboratories**
**Kawasaki Steel Corporation 1, Kawasaki-cho**
**Chiba-shi Chiba (JP)**
Inventor: **Matsumura, Yasuharu c/o Research Laboratories**
**Kawasaki Steel Corporation 1, Kawasaki-cho**
**Chiba-shi Chiba (JP)**
Inventor: **Harimaya, Senichi c/o Research Laboratories**
**Kawasaki Steel Corporation 1, Kawasaki-cho**
**Chiba-shi Chiba (JP)**
Inventor: **Kamino, Yoshikazu c/o Research Laboratories**
**Kawasaki Steel Corporation 1, Kawasaki-cho**
**Chiba-shi Chiba (JP)**
Inventor: **Kitaoka, Hidenari c/o Research Laboratories**
**Kawasaki Steel Corporation 1, Kawasaki-cho**
**Chiba-shi Chiba (JP)**

(74) Representative: **Henkel, Feiler, Hänzel & Partner**
**Möhlstrasse 37**
**D-8000 München 80 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

(56) References cited:

CHEMICAL ABSTRACTS, vol. 40, 1946, column 7065-9, Columbus, Ohio, US; N.D. IVANOVA et al.: "Colorimetric determination of phosphorus in iron alloys" & ZAVODSKAYA LAB. 12, 246-8(1946)

## Description

Background of the invention

This invention relates to a test medium and method for detecting phosphorus segregates, and more particularly, to such a method capable of rapidly and easily detecting the distribution of phosphorus in metallic materials such as continuously cast steel slabs and large-sized steel ingots.

Heretofore, segregation in large-sized steel ingots has been judged by sulfur printing. This method is by attaching photographic paper impregnated with aqueous sulfuric acid to a polished cross section of a large-sized steel ingot, thereby detecting hydrogen sulfide given off from segregated sulfur as stains on the photographic paper. This method has been widely used on the production line. Recently, however, steels subjected to low sulfur treatment and Ca treatment, such as steels resistant to hydrogen embrittlement cracking, have been put into practical use, and much progress has been made in the art to manufacture high purity steel and to prevent segregation in continuous castings. Such advanced steels having extremely low sulfur contents are difficult to test for solidification segregates by the conventional sulphur printing.

Aside from the sulfur printing, a macroanalyzer is known as a device for examining the segregation of alloying elements. The macroanalyzer can quantitatively evaluate a planar section of a large-sized steel ingot by applying an electron beam to the section and detecting the spectrum of X-rays generated as in EPMA. However, this method is not applicable to a commercial production process because it uses an expensive device, the surface to be examined must be finished by emery paper of the order of #1,000, the measurement of a sample takes more than one hour, the configuration of a sample is limited, it cannot be applied to a wide section sample, and so on.

One known method of detecting phosphorus is the phosphorus printing reported by M. Niessner in 1932. This method is by attaching a test paper which has been immersed in liquid B shown below in Table 1 to a surface of steel to be examined for 3—5 minutes, removing the paper from the steel surface, and thereafter dipping the test paper into liquid A for 3—4 minutes, thereby producing a printed image. Because of unclear printed images and low sensitivity, it is difficult to detect phosphorus segregates by this method in commercial grade steels. Also It requires an additional procedure of dipping the test paper in another liquid after removal from a steel surface.

TABLE 1

| | |
|---|---|
| Liquid A | |
| Stannous chloride saturated solution | 5 ml |
| Concentrated hydrochloric acid | 50 ml |
| Water | 100 ml |
| Alum | minor amount |
| Liquid B | |
| Ammonium molybdate | 5 g |
| Water | 100 ml |
| Nitric acid (specific gravity 1.2) | 35 ml |

Furthermore in C. A. *41* (1947), ol. 6171 d-f and C. A. *40* (1946), col. 7065-9 there is described the colorimetric determination of phosphorus in steel or iron alloys on the basis of Ammonium molybdate.

Summary of the invention

It is an object of the present invention to provide a novel improved test medium and method capable of rapidly detecting segregates in metallic materials such as Ca-loaded steels and low-sulfur steels over a large surface area as easily as by the sulfur printing. In this method, the element to be detected in place of sulfur is phosphorus, which has the great likelihood to segregate upon freezing, and phosphorus segregates are detected on test paper as stains.

It is therefore, another object of the present invention to provide a novel method for detecting phosphorus segregation which takes the place of the conventional phosphorus printing method, can produce a clear printed image with high sensitivity through an easy printing operation, and is suitable for use in the control of an in-place production process.

According to a first aspect of the present invention, there is provided the use of an aqueous solution containing 0.1 to 80% by weight of silver nitrate for detecting phosphorus segregates in a metallic material.

According to a second aspect of the present invention, the solution further comprises a compatible amount of an alcohol.

According to a third aspect of the present invention, there is provided the use of a sheet impregnated with an aqueous solution containing 0.1 to 80% by weight of silver nitrate (for detecting phosphorus segregates in a metallic material.)

According to a fourth aspect, the solution further comprises a compatible amount of an alcohol.

According to a fifth aspect of the present invention, there is provided the use of a sheet having an

effective amount of silver nitrate incorporated therein in a dry state for detecting phosphorus segregates in a metallic material.

According to a sixth aspect of the present invention, there is provided a method for detecting phosphorus segregates in a metallic material, comprising

(a) attaching a test sheet onto that surface of a metallic material to be tested,

(b) maintaining the sheet in contact with the metallic material surface in the presence of an aqueous solution comprising 0.1 to 80% by weight of silver nitrate for a sufficient time, and

(c) removing the sheet from the metallic material surface.

According to a seventh aspect, there is provided a method for detecting phosphorus segregates in a metallic material, comprising

(a) attaching a test sheet onto that surface of a metallic material to be tested,

(b) maintaining the sheet in contact with the metallic material surface in the presence of an aqueous solution comprising 0.1 to 80% by weight of silver nitrate and an alcohol for a sufficient time, and

(c) removing the sheet from the metallic material surface, the sheet having an image of segregates developed.

According to an eighth aspect, the method as defined above further comprises

(d) subjecting the sheet from step (c) to fixing treatments.

According to a nineth aspect, the method as defined above further comprises etching the surface of the metallic surface to be tested prior to step (a).

The term metallic materials used herein generally designates various steels.

## Brief description of the drawings

The invention will be more fully understood when taken in conjunction with the accompanying drawings, in which:

Figures 1 to 3 are photographic phosphorus prints representing phosphorus segregates in a continuously cast steel billet;

Figures 4 to 7 are similar photographic phosphorus prints representing phosphorus segregates in another continuously cast steel billet;

Figures 8 and 9 are microanalyzer photographs showing segregated phosphorus patterns in regions corresponding to those shown in Figures 1—3 and Figures 4—7, respectively;

Figures 10a and 11a are photographic phosphorus prints showing phosphorus segregates in continuously cast billets according to the present invention; and

Figures 10b and 11b are macroanalyzer photographs showing phosphorus segregates in the same billets.

## Detailed description of the invention

The principle of the present invention will be briefly described. Like sulfur, phosphorus has the great likelihood of segregating upon solidification and is thus concentrated at the finally solidified region. Phosphorus rich portions are lower in electrochemical series and preferentially dissolved in etching solution. Ogura et al. reported in Journal of Japanese Metallurgy Associate, *45*, 10, 1093 (1981), that the depth of furrows at grain boundary in steel etched with picric acid etchant is in quantitative relationship to the quantity of phosphorus segregated at grain boundary.

One of the inventors reported that when a steel specimen is anodized in an electrolyte based on an alcohol, phosphorus present in a solid solution form in the steel is converted to a phosphorus-containing oxo acid as the matrix is dissolved, which in turn, reacts with eluted iron ion to form an Fe—P—O—H compound (see Japanese Patent Application No. 58—119853 i.e. JP—A—60—111541). The present invention makes use of such an Fe—P—O—H compound to detect phosphorus segregates in cast steel. Fe—P—O—H compounds are difficultly soluble in alcohol and deposit simultaneously with their formation at the site of eluted phosphorus in steel. When the steel is gently washed by alcohol and air dried with a dryer, air exposure causes the Fe—P—O—H compound which has deposited at the site of phosphorus segregates to automatically give rise to redox reaction so that it is decomposed to give off phosphine. The principle of the present invention is based on the detection of phosphine. It is believed that phosphine is given off when an oxo acid having a low oxidation number is decomposed as shown in the following formula.

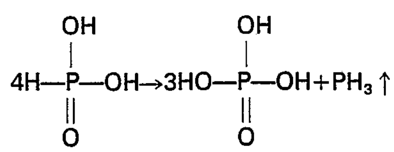

It is thus essential for the phosphorus in solid solution to be converted into an oxo acid having a low oxidation number as the matrix is dissolved. This conversion is promoted by an alcohol used as the solvent of etching solution. The amount of the Fe—P—O—H compound formed largely depends upon the nature of the etching solution, and is increased as the concentration of acid in the solution is increased, and when the

etching solution is free of a complexing agent capable of forming a complex with iron ion, or if present, as the complexing ability of the agent is reduced.

The inventors have found that by virtue of the preferential etching of phosphorus concentrated portions, phosphorus segregated on a solidified steel slab can be readily detected in a short time by introducing an aqueous solution containing 0.1 to 80% by weight of silver nitrate ($AgNO_3$) and optionally, an alcohol between that surface of the steel to be tested and a test sheet on which a pattern of phosphorus segregation is printable.

The medium for detecting segregated phosphorus may vary in form. According to a first embodiment to carry out the method of the present invention, the detecting medium is in the form of an aqueous solution containing 0.1 to 80% by weight of silver nitrate ($AgNO_3$) and optionally, an alcohol. On use, a dry sheet or coupon is attached onto the surface of steel to be tested and the aqueous solution is applied to the sheet as by spraying, brushing or coating to cause the solution to reach the steel surface.

According to a second embodiment of the present invention, the detecting means are used in the form of a wet sheet or coupon, that is, sheet or coupon impregnated or coated with the above-mentioned aqueous solution. The wet sheet, which has contained silver nitrate and an optional alcohol in a proper amount, is ready for use, that is, it is simply attached or pressed to the surface of steel to be tested. In some cases, the test sheet may be coated with the solution immediately before it is pressed to the steel surface.

According to a third embodiment of the present invention, the detecting means are used in the form of a dry sheet or coupon having silver nitrate borne thereon in a dry state. This dry sheet is prepared by impregnating a sheet with an aqueous solution of silver nitrate followed by drying. The dry sheet is used by attaching it to the surface of steel to be tested, and applying a suitable amount of water to the sheet such that an aqueous solution containing 0.1 to 80% by weight of silver nitrate is present between the steel surface and the sheet.

In any embodiment, it is necessary that an aqueous solution containing 0.1 to 80% by weight of silver nitrate contacts the surface of steel to be tested. Solutions containing less than 0.1% by weight of silver nitrate attack the steel too weakly to detect segregated phosphorus because stains or discolored spots on a print is blurred whereas concentrations of higher than 80% by weight result in deposits of the salt on the sheet to render it unuseful.

The alcohols which are optionally contained in the silver nitrate solution may be any desired alcohols, for example, methanol, ethanol, and propanol as long as they are liquid at room temperature. The concentration of alcohol in the aqueous silver nitrate solution is not particularly limited as long as the alcohol is compatible with the silver nitrate in the solution in the above-specified concentrations.

The sheets used in these embodiments may be any desired sheet-like articles of materials capable of bearing silver nitrate such as wood and synthetic resins, and preferably paper.

The test sheets are attached to the surface of steel to be tested and maintained in contact with the steel surface for several minutes, for example, 3 to 10 minutes. The sheets having an image of segregates developed thereon are then removed from the steel surface and immersed in an aqueous solution of about 10 to 30% by weight of sodium thiosulfate for several minutes, for example, 5 minutes for fixing. The thus treated sheets are rinsed with running water and dried. The resulting sheets are prints clearly showing spots of segregated phosphorus as black spots.

It has been found that the silver nitrate solution with or without an alcohol serves as an etchant as well as a color producing reagent through reduction of silver. Since silver nitrate is weaker in attacking or etching action than ordinary acids, phosphorus segregated portions which are lower in electrochemical series are preferentially dissolved and silver ions are reduced and precipitated in cooperation with the dissolution of the iron matrix, enabling detection of segregated phosphorus.

As understood from the above-mentioned principle of the present invention, the surface of steel to be tested for the presence of segregated phosphorus may preferably be etched prior to the above-described detecting process. The etching solutions used for the previous etching may be solutions containing at least one of mineral acids, organic acids and salts thereof, and an alcohol. Once the surface of steel to be tested is attacked by such an etching solution, the etching solution is removed and the steel surface is subjected to the above-described testing process.

Examples of the acids include mineral acids such as hydrochloric acid, sulfuric acid, perchloric acid, phosphoric acid and nitric acid; organic acids, such as picric acid, salicylic acid, sulfosalicylic acid, acetic acid, formic acid, lactic acid and malic acid; and salts such as lithium chloride, copper chloride, calcium chloride, zinc chloride, iron chloride, aluminum chloride, copper sulfate, copper nitrate and tetramethyl ammonium chloride. The alcohols which promote the attack on metal by acid may be selected from the same group as mentioned for the silver nitrate solution. The concentrations of acid and alcohol in the etching solution may vary with the characteristics of the steel surface to be tested including phosphorus concentration and the only requirement is that the alcohol is compatible with the acid in the solution.

When the steel surface to be tested is previously attacked by such a pre-etching solution, the etchant reacts with segregated phosphorus to form difficultly soluble phosphorus compounds. Upon removal of the pre-etching solution, the difficultly soluble phosphorus compounds are exposed to the ambient atmosphere to give off phosphine ($PH_3$) gas.

The silver nitrate solution is applied to the thus pre-treated surface. In the case of solutions having lower concentrations of silver nitrate above 0.1%, silver cations are reduced by $PH_3$ into metallic silver

which appears as black spots or stains representing segregated phosphorus. In the case of solutions having higher concentration of silver nitrate below 80%, silver cations are directly reduced by the matrix iron to exhibit black color as in the case where the pre-etching is eliminated.

Examples of the present invention are presented below by way of illustration and not by way of limitation.

Example 1

A steel specimen was sectioned from a segregated region of a continuously cast slab of ordinary carbon steel having a phorphorus content of 0.02% by weight. It was polished with #400 emery paper and fully cleaned with absorbent wadding. A wet test paper coupon which was impregnated with an aqueous solution of 10% by weight of silver nitrate and 50% by volume of ethanol was attached to the surface of the specimen to be tested and maintained in pressure contact for 5 minutes. The test paper having an image of segregates developed was removed from the specimen surface and fixed with an aqueous solution of 10% by weight of sodium thiosulfate. This fixing procedure is the same for the following examples. There was obtained a printed image as shown in Figure 1.

Example 2

A steel specimen was taken out, polished, and cleaned in the same manner as in Example 1. A dry test paper coupon containing 6 grams of silver nitrate per square meter was attached to the surface of the specimen to be tested. Absorbent wadding full of water was forced to and moved throughout the paper such that the test paper was fully wetted with an aqueous solution of 0.1 to 80% by weight of silver nitrate. The paper was maintained in pressure contact with the specimen surface for 9 minutes. The paper was removed and fixed. There was obtained a printed image as shown in Figure 2.

Example 3

A steel specimen was taken out, polished, and cleaned in the same manner as in Example 1. A test paper coupon free of any agent was attached to the surface of the specimen to be tested. Absorbent wadding full of an aqueous solution containing 5% by weight of silver nitrate and 50% by volume of methanol was forced to and moved throughout the paper to fully wet the paper. The paper was maintained in pressure contact with the specimen surface for 7 minutes. The paper was removed and fixed. There was obtained a printed image as shown in Figure 3.

Example 4

A freshly sectioned surface of a segregated region of a continuously cast slab of ordinary carbon steel having a phosphorus content of 0.02% by weight was polished with #180 emery paper and fully cleaned with absorbent wadding wetted with ethanol. The specimen was immersed in a 5 vol% hydrochloric acid/ethanol solution for 5 minutes for etching. The etched specimen was fully cleaned with an alcohol and then a test sheet wetted with an aqueous solution containing 5% by weight of silver nitrate and 50% by volume of propanol was attached to the specimen for 3 minutes. The test sheet was removed and fixed. There was obtained a printed image as shown in Figure 4.

Example 5

A freshly sectioned surface of a segregated region of a continuous cast slab of ordinary carbon steel having a phosphorus content of 0.02% by weight was polished with #180 emery paper and fully cleaned with absorbent wadding wetted with ethanol. The specimen was immersed in a saturated picric acid/ethanol solution for 5 minutes for etching. The etched specimen was fully cleaned with ethanol and then a dry test sheet containing 6 grams per square meter of silver nitrate was attached to the specimen. Water was sprayed onto the sheet to an amount of about 150 ml per square meter and the sheet was maintained in contact with the specimen surface for 3 minutes. The test sheet was removed and fixed. There was obtained a printed image as shown in Figure 5.

Example 6

A freshly sectioned surface of a segregated region of a continuously cast slab of ordinary carbon steel having a phosphorus content of 0.02% by weight was polished with #180 emery paper and fully cleaned with absorbent wadding wetted with ethanol. The specimen was immersed in an ethanol solution of 5% by weight ferric chloride for 5 minutes for etching. The etched specimen was fully cleaned with ethanol and then a test sheet was attached to the specimen and wetted with an aqueous solution of 5% by weight silver nitrate and the sheet was maintained in contact with the specimen surface for 3 minutes. The test sheet was removed and fixed. There was obtained a printed image as shown in Figure 6.

Example 7

A freshly sectioned surface of a segregated region of a continuously cast slab of ordinary carbon steel having a phosphorus content of 0.02% by weight was polished with #180 emery paper and fully cleaned with absorbent wadding wetted with ethanol. The specimen was immersed in an ethanol solution of 4% by weight salicylic acid and 2% by weight of lithium chloride for 5 minutes for etching. The etched specimen

was fully cleaned with ethanol and then a test sheet wetted with an aqueous solution of 5% by weight of silver nitrate was attached to the specimen and maintained in contact with the specimen surface for 3 minutes. The test sheet was removed and fixed. There was obtained a printed image as shown in Figure 7.

Figures 8 and 9 are photographs taken on the steel slabs used in Examples 1—3 and Examples 4—7, respectively, by means of a macroanalyzer, illustrating the distribution of phosphorus thereon. With respect to the phosphorus distribution pattern on a cross section of cast steel, the printed images shown in Figures 1—3 and 4—7 conform to the macroanalyzer photographs of Figures 8 and 9, proving that the present invention is fully effective in detecting phosphorus segregation. It should be noted that the prints of Figures 1—3 are the mirror images of the macroanalyzer photograph of Figure 8, and the prints of Figures 4—7 are the mirror images of the macroanalyzer photograph of Figure 9.

Example 8

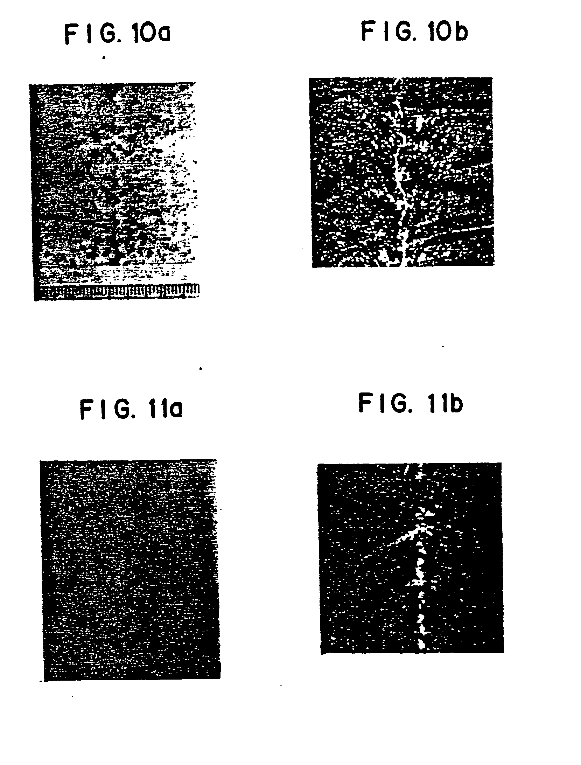

A section of a segregated part of a continuously cast billet of ordinary carbon steel containing 0.02% P was polished with #240 emery paper and cleaned with cotton wadding soaked with ethanol. The cleaned surface was etched and printed under the conditions shown in Table 2, and the color development examined. The results for two different types of billets are shown in Figures 10a and 11a.

TABLE 2
Phosphorus printing conditions

| | |
|---|---|
| Etching conditions | |
| Etching liquid: | 10 vol% acetylacetone, 3 wt% thenoyltrifluoroacetone, and 1 wt% lithium chloride in ethanol |
| Temperature: | room temperature |
| Time: | 2 minutes |
| Printing conditions | |
| Color producing solution: | 5 wt% $AgNO_3$ in a 1:1 mixture of water and ethanol |
| Contact time: | 1 minute |

Figures 10b and 11b are the measurements of the phosphorus distribution on the two different billets by a macroanalyzer. The prints of phosphorus distribution shown in Figures 10a and 11a well conforms to the macroanalyzer measurements shown in Figures 10b and 11b, proving that this invention is sufficiently effective in detecting segregated phosphorus. It is to be noted that phosphorus prints as shown in Figures 10a and 11a are mirror images of macroanalyzer measurements as shown in Figures 10b and 11b, respectively.

As seen from the foregoing examples, the present invention allows segregated phosphorus to be detected within several minutes after the polishing of specimens without limitation on the size and configuration of steel products to be tested. When pre-etching is employed, the method of the present invention can detect phosphorus segregates in a shorter time, i.e., two minutes of etching time and one minute of printing time. It has been found that this invention allows phosphorus to be adequately detected even at concentrations as low as 30 parts per million parts of the matrix. Furthermore, unlike the sulfur printing, it eliminates troublesome operation in a dark room. Since no particular installation is needed for the detecting process because of the elimination of generation of any deleterious gases, the practice of the present invention is very easy during continuous steel casting in actual works. The present invention is thus very useful and benefitable in steel making. It is also very convenient that prints showing segregated phosphorus can be stored as records.

**Claims**

1. Use of an aqueous solution containing 0.1 to 80% by weight of silver nitrate for detecting phosphorus segregates in a metallic material.
2. Use of a solution according to claim 1 wherein the solution further comprises a compatible amount of an alcohol.
3. Use of a solution according to claim 2 wherein the solution comprises as the alcohol a lower alkyl alcohol and as methanol, ethanol and propanol.
4. Use of a sheet impregnated with an aqueous solution according to claim 1 containing 0.1 to 80% by weight of silver nitrate for detecting phosphorus segregates in a metallic material.
5. Use of a sheet according to claim 4 wherein the solution further comprises a compatible amount of an alcohol.
6. Use of a sheet according to claim 5 wherein the alcohol is selected from lower alkyl alcohols.
7. Use of a sheet according to claim 4 wherein the sheet is paper.

8. Use of a sheet according to claim 1 having an effective amount of silver nitrate incorporated therein in a dry state for detecting phosphorus segregates in a metallic material.

9. Use of a sheet according to claim 8 wherein silver nitrate is incorporated in the sheet by impregnating the sheet with an aqueous solution of silver nitrate followed by drying.

10. Use of a sheet according to claim 8 wherein the sheet is paper.

11. A method for detecting phosphorus segregates in a metallic material, comprising

(a) attaching a test sheet as used according to claims 4—10 onto that surface of a metallic material to be tested,

(b) maintaining the sheet in contact with the metallic material surface in the presence of an aqueous solution comprising 0.1 to 80% by weight of silver nitrate for a sufficient time, and

(c) removing the sheet from the metallic material surface.

12. The method according to claim 11 wherein the metallic material is steel.

13. The method according to claim 11 wherein the silver nitrate solution further contains an alcohol.

14. The method according to claim 13 wherein the alcohol is selected from lower alkyl alcohols such as methanol, ethanol and propanol.

15. The method according to claim 11 which further comprises

(d) subjecting the sheet from step (c) to fixing treatments.

16. The method according to claim 11 wherein step (b) includes (b-1) applying an aqueous silver nitrate solution to the sheet and (b-2) maintaining the sheet in contact with the metallic material surface.

17. The method according to claim 11 wherein the test sheet has been impregnated with an aqueous silver nitrate solution prior to step (a).

18. The method according to claim 11 wherein the test sheet has silver nitrate borne therein in a dry state and step (b) includes (b-1) applying water to the sheet and (b-2) maintaining the sheet in contact with the metallic material surface.

19. The method according to claim 11 which further comprises

etching the surface of the metallic surface to be tested prior to step (a).

20. The method according to claim 19 wherein the etching is carried out using an etching solution comprising an acid and an alcohol.

21. The method according to claim 20 wherein the acid is selected from mineral acids, organic acids, and salts thereof.

22. The method according to claim 20 wherein the alcohol is selected from lower alkyl alcohol such as methanol, ethanol and propanol.

23. The method according to claim 15 wherein after removal from the metallic surface, the test sheet is immersed in a solution of sodium thiosulfate for a sufficient time to fix the printed image.

**Patentansprüche**

1. Verwendung einer wäßrigen Lösung mit 0,1—80 Gew.-% Silbernitrat zum Nachweis von Phosphorabscheidungen in einem metallischen Material.

2. Verwendung einer Lösung nach Anspruch 1, die zusätzlich eine verträgliche Menge eines Alkohols enthält.

3. Verwendung einer Lösung nach Anspruch 2, die als Alkohol einen kurzkettigen Alkylalkohol, wie Methanol, Ethanol und Propanol, enthält.

4. Verwendung einer mit einer wäßrigen Lösung nach Anspruch 1 bis 0,1—80 Gew.-% Silbernitrat imprägnierten Lage zum Nachweis von Phosphorabscheidungen in einem metallischen Material.

5. Verwendung einer Lage nach Anspruch 4, die mit einer eine verträgliche Menge eines Alkohols enthaltenden Lösung imprägniert ist.

6. Verwendung einer Lage nach Anspruch 5, wobei der Alkohol aus niedrigen Alkylalkoholen ausgewählt ist.

7. Verwendung einer Lage nach Anspruch 4, die aus Papier besteht.

8. Verwendung einer Lage nach Anspruch 1 mit einer in trockenem Zustand einverleibten wirksamen Menge Silbernitrat zum Nachweis von Phosphorabscheidungen in einem metallischen Material.

9. Verwendung einer Lage nach Anspruch 8, in die das Silbernitrat durch Imprägnieren der Lage mit einer wäßrigen Silbernitratlösung und anschließendes Trocknen einverleibt ist.

10. Verwendung einer Lage nach Anspruch 8, die aus Papier besteht.

11. Verfahren zum Nachweis von Phosphorabscheidungen in einem metallischen Material, gekennzeichnet durch

(a) Anbringen einer gemäß Ansprüchen 4 bis 10 verwendbaren Testlage an der zu testenden Oberfläche eines metallischen Materials;

(b) ausreichend langes Inberührungshalten der Lage mit der metallischen Materialoberfläche in Gegenwart einer wäßrigen Lösung mit 0,1—80 Gew.-% Silbernitrat und

(c) Entfernen der Lage von der metallischen Materialoberfläche.

12. Verfahren nach Anspruch 11, dadurch gekennzeichnet, daß das metallische Material aus Stahl besteht.

13. Verfahren nach Anspruch 11, dadurch gekennzeichnet, daß die Silbernitratlösung zusätzlich einen Alkohol enthält.

14. Verfahren nach Anspruch 13, dadurch gekennzeichnet, daß der Alkohol aus kurzkettigen Alkylalkoholen, wie Methanol, Ethanol und Propanol, ausgewählt ist.

15. Verfahren nach Anspruch 11, dadurch gekennzeichnet, daß zusätzlich die Lage aus Stufe (c) einer Fixierbehandlung unterworfen wird.

16. Verfahren nach Anspruch 11, dadurch gekennzeichnet, daß die Stufe (b) aus (b-1) einer Applikation einer wäßrigen Silbernitratlösung auf die Lage und (b-2) Inberührunghalten der Lage mit der metallischen Materialoberfläche besteht.

17. Verfahren nach Anspruch 11, dadurch gekennzeichnet, daß die Testlage vor Durchführung der Stufe (a) mit einer wäßrigen Silbernitratlösung imprägniert wurde.

18. Verfahren nach Anspruch 11, dadurch gekennzeichnet, daß die Testlage Silbernitrat in trockenem Zustand enthält und daß die Stufe (b) aus (b-1) einer Applikation von Wasser auf die Lage und (b-2) Inberührunghalten der Lage mit der metallischen Materialoberfläche besteht.

19. Verfahren nach Anspruch 11, dadurch gekennzeichnet, daß zusätzlich vor Durchführung der Stufe (a) die metallische Oberfläche oberflächlich geätzt wird.

20. Verfahren nach Anspruch 19, dadurch gekennzeichnet, daß mit Hilfe einer eine Säure und einen Alkohol enthaltenden Ätzlösung geätzt wird.

21. Verfahren nach Anspruch 20, dadurch gekennzeichnet, daß die Säure aus Mineralsäuren, organischen Säuren und deren Salzen ausgewählt ist.

22. Verfahren nach Anspruch 20, dadurch gekennzeichnet, daß der Alkohol aus niedrigen Alkylalkoholen, wie Methanol, Ethanol und Propanol, ausgewählt ist.

23. Verfahren nach Anspruch 15, dadurch gekennzeichnet, daß die Testlage nach Entfernen von der metallischen Oberfläche ausreichend lange zur Fixierung der Bildkopie in eine Natriumthiosulfatlösung getaucht wird.

**Revendications**

1. Utilisation d'une solution aqueuse contenant 0,1 à 80% en poids de nitrate d'argent pour la détection des ségrégats de phosphore dans un matériau métallique.

2. Utilisation d'une solution selon la revendication 1, dans laquelle la solution comprend en outre une quantité compatible d'un alcool.

3. Utilisation d'une solution selon la revendication 2, dans laquelle la solution comprend, à titre d'alcool, un alcool alkylique inférieur tel que le méthanol, l'éthanol et le propanol.

4. Utilisation d'une feuille imprégnée avec une solution aqueuse selon la revendication 1, qui contient 0,1 à 80% en poids de nitrate d'argent pour la détection des ségrégats de phosphore dans un matériau métallique.

5. Utilisation d'une feuille selon la revendication 4, dans laquelle la solution comprend en outre une quantité compatible d'un alcool.

6. Utilisation d'une feuille selon la revendication 5, dans laquelle on choisit l'alcool parmi les alcools alkyliques inférieurs.

7. Utilisation d'une feuille selon la revendication 4, dans laquelle la feuille est du papier.

8. Utilisation d'une feuille selon la revendication 1, qui possède une quantité efficace de nitrate d'argent incorporée en elle à l'état sec, pour la détection des ségrégats de phosphore dans un matériau métallique.

9. Utilisation d'une feuille selon la revendication 8, dans laquelle on incorpore le nitrate d'argent dans la feuille par imprégnation de la feuille avec une solution aqueuse de nitrate d'argent, suivie d'un séchage.

10. Utilisation d'une feuille selon la revendication 8, dans laquelle la feuille est du papier.

11. Procédé pour la détection des ségrégats de phosphore dans un matériau métallique, qui comprend

(a) la fixation d'une feuille d'essai telle qu'utilisée conformément aux revendications 4—10, sur la surface d'un matériau métallique à tester,

(b) le maintien de la feuille en contact avec la surface du matériau métallique, en présence d'une solution aqueuse comprenant 0,1 à 80% en poids de nitrate d'argent pendant un temps suffisant, et

(c) le retrait de la feuille de la surface du matériau métallique.

12. Procédé selon la revendication 11, dans lequel le matériau métallique est de l'acier.

13. Procédé selon la revendication 11, dans lequel la solution de nitrate d'argent contient en outre un alcool.

14. Procédé selon la revendication 13, dans lequel on sélectionne l'alcool parmi les alcools alkyliques inférieurs tels que le méthanol, l'éthanol et le propanol.

15. Procédé selon la revendication 11, qui comprend en outre

(d) la soumission de la feuille provenant de l'étape (c) à des traitements de fixation.

16. Procédé selon la revendication 11, dans lequel l'étape (b) englobe, (b-1) l'application d'une solution aqueuse de nitrate d'argent à la feuille et (b-2) le maintien de la feuille en contact avec la surface du matériau métallique.

17. Procédé selon la revendication 11, dans lequel on a imprégné la feuille d'essai avec une solution aqueuse de nitrate d'argent avant l'étape (a).

18. Procédé selon la revendication 11, dans lequel la feuille d'essai possède du nitrate d'argent fixé sur elle à l'état sec, et l'étape (b) englobe (b-1) l'application d'eau sur la feuille et (b-2) le maintien de la feuille en contact avec la surface du matériau métallique.

19. Procédé selon la revendication 11, qui comprend en outre l'attaque de la surface du matériau métallique à examiner avant l'étape (a).

20. Procédé selon la revendication 19, dans lequel on effectue l'attaque à l'aide d'une solution décapante comprenant un acide et un alcool.

21. Procédé selon la revendication 20, dans lequel on choisit l'acide parmi les acides minéraux, les acides organiques et leurs sels.

22. Procédé selon la revendication 20, dans lequel on choisit l'alcool parmi les alcools alkyliques inférieurs tels que le méthanol, l'éthanol et le propanol.

23. Procédé selon la revendication 15, dans lequel après sont retrait de la surface métallique, on immerge la feuille d'essai dans une solution de thiosulfate de sodium pendant un temps suffisant afin de fixer l'image imprimée.

FIG. 1

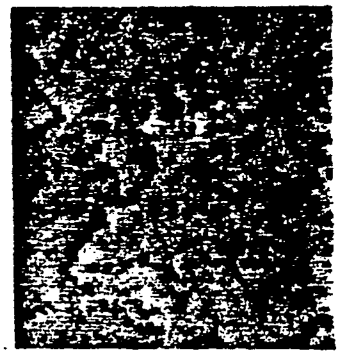

FIG. 2

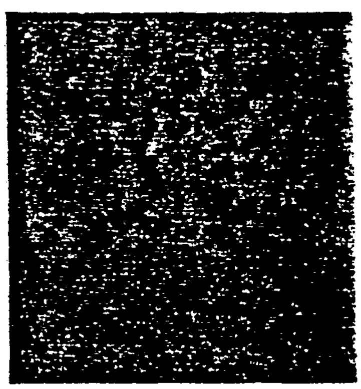

FIG. 3

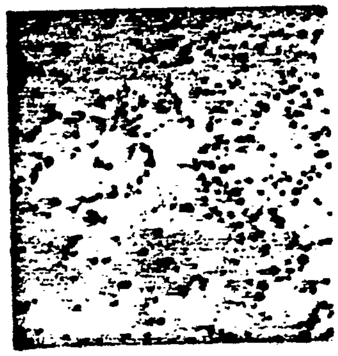

FIG. 4

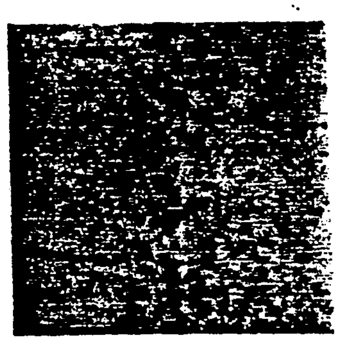

FIG. 5

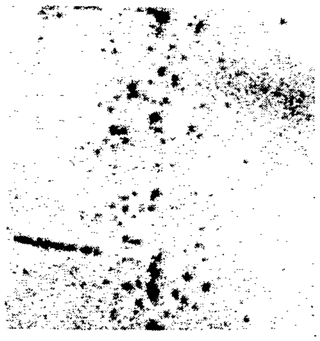

FIG. 6

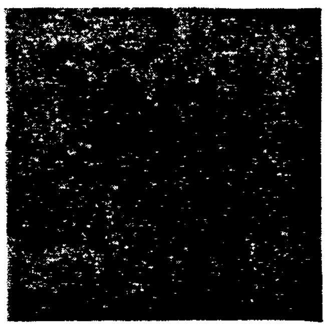

FIG. 7

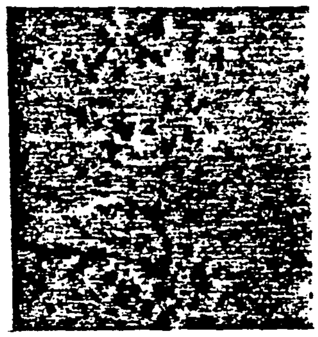

FIG. 8

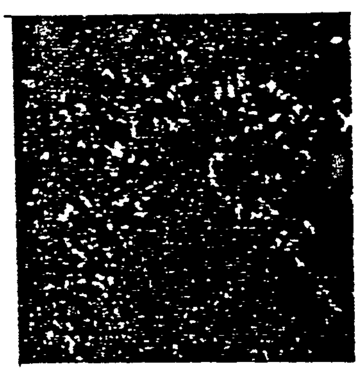

FIG. 9

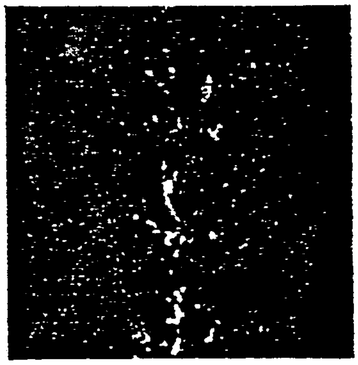

FIG. 10a

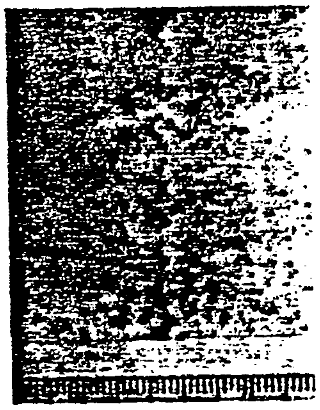

FIG. 10b

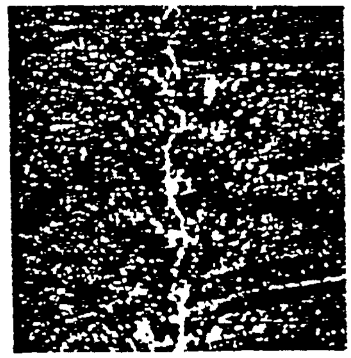

FIG. 11a

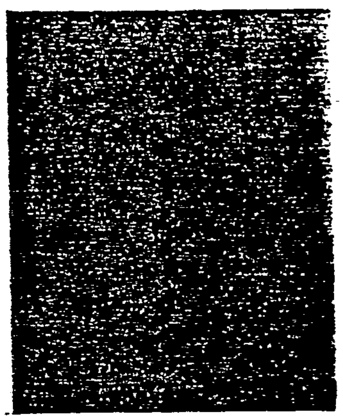

FIG. 11b